# EUROPÄISCHE PATENTSCHRIFT

(11) **EP 2 632 930 B1**
(45) Veröffentlichungstag und Bekanntmachung des Hinweises auf die Patenterteilung: **09.03.2016**
(21) Anmeldenummer: 11785276.4
(22) Anmeldetag: 28.10.2011
(51) Int. Cl.: C07G 1/00, D21C 11/00, C08H 7/00, C08L 97/02, C08H 8/00

(54) **VERFAHREN ZUR GEWINNUNG VON LIGNIN**
METHOD FOR PRODUCTION OF LIGNIN
PROCÉDÉ D'EXTRACTION DE LIGNINE

(30) Priorität: 29.10.2010 AT 17992010
(43) Veröffentlichungstag der Anmeldung: 04.09.2013
(73) Patentinhaber: Annikki GmbH, 8020 Graz (AT)
(72) Erfinder: TERS, Thomas, A-1230 Wien (AT); FUQAHA, Abdel Halim, A-3430 Tulln (AT); FRIEDL, Anton, A-1130 Wien (AT); SREBOTNIK, Ewald, A-2352 Gumpoldskirchen (AT); ZIKELI, Florian, A-7100 Neusiedl am See (AT); MESSNER, Kurt, A-1170 Wien (AT)
(74) Vertreter: Schwarz, Albin
(86) Internationale Anmeldenummer: PCT/AT2011/000438
(87) Internationale Veröffentlichungsnummer: WO 2012/054947

(56) Entgegenhaltungen:
- EP-A1- 0 498 330
- EP-A2- 0 140 226
- DE-A1- 4 308 951
- US-A- 3 585 104
- US-A- 5 777 086
- G. Dahlmann: "Pulping of spruce and pine with alcohol and alkali by the organocell process", TAPPI Proceedings, 1. Januar 1990 (1990-01-01), Seiten 657-661, XP55028139, Gefunden im Internet: URL:http://www.tappi.org/Downloads/unsorte d/UNTITLED---pulp90657pdf.aspx [gefunden am 2012-05-24]
- DAHLMANN G ET AL: "THE ORGANOCELL PROCESS-PULPING WITH THE ENVIRONMENT IN MIND", TAPPI JOURNAL, TECHNICAL ASSOCIATION OF THE PULP & PAPER INDUSTRY. ATLANTA, US, Bd. 73, Nr. 4, 1. April 1990 (1990-04-01), Seiten 237-240, XP000133885, ISSN: 0734-1415
- ZHAO X ET AL: "Organosolv pretreatment of lignocellulosic biomass for enzymatic hydrolysis", APPLIED MICROBIOLOGY AND BIOTECHNOLOGY, SPRINGER, BERLIN, DE, Bd. 82, Nr. 5, 1. April 2009 (2009-04-01), Seiten 815-827, XP002609940, ISSN: 1432-0614, DOI: 10.1007/S00253-009-1883-1 [gefunden am 2009-02-12]
- GHATAK ET AL: "Spectroscopic comparison of lignin separated by electrolysis and acid precipitation of wheat straw soda black liquor", INDUSTRIAL CROPS AND PRODUCTS, ELSEVIER, NL, Bd. 28, Nr. 2, 1. September 2008 (2008-09-01), Seiten 206-212, XP023178276, ISSN: 0926-6690, DOI: 10.1016/J.INDCROP.2008.02.011 [gefunden am 2008-04-09]
- XIONG ET AL: "Application of Brown-Rot Basidiomycete Fomitopsis sp. IMER2 for Biological Treatment of Black Liquor", JOURNAL OF BIOSCIENCE AND BIOENGINEERING, ELSEVIER, AMSTERDAM, NL, Bd. 104, Nr. 6, 1. Dezember 2007 (2007-12-01), Seiten 446-450, XP022428324, ISSN: 1389-1723, DOI: 10.1263/JBB.104.446

## Beschreibung

Als Reaktion auf schwindende Ölvorräte und mögliche Unsicherheiten in der Ölversorgung haben Bioraffinerie Prozesse stark an Bedeutung gewonnen. Darunter versteht man die Zerlegung von Biomasse in ihre einzelnen Komponenten und deren Weiterverarbeitung zu höherwertigen Produkten. Der wirtschaftlichste Rohstoff ist Lignocellulose wie Holz oder Einjahrespflanzen (z.B. Getreidestroh), die in großen Mengen verfügbar sind und nicht in Konkurrenz zu Nahrungsmitteln stehen. Ein wesentlicher ökonomischer Faktor bei deren Nutzung ist das sogenannte Aufschlussverfahren ("Pre-treatment"). Ein ideales Aufschlussverfahren zerlegt Lignocellulose ohne hohen Energieaufwand möglichst selektiv in seine Hauptkomonenten Cellulose, Hemicelulose und Lignin. Im Speziellen das Lignin hat sich als möglicher Ersatz für petrochemische Rohstoffe zu einem begehrten Ausgangsstoff für die chemische Industrie entwickelt und stellt z.B. Anwendungen in niedermolekularer Form als Treibstoffkomponenten, Klebstoffe, Bindemittel oder in höhermolekularer Form als Polymerersatz, bzw. -zusatz, Harzkomponenten, Carbonfasem etc. in Aussicht. Die Art des Aufschlussprozesses beeinflusst dabei wesentlich die Eigenschaften des gewonnenen Lignins, wie auch das Downstream Processing der Ligninfraktion. Die meisten Aufschlussprozesse erfolgen bei Temperaturen zwischen 100° und 200°C, enthalten thermische Abbau- und Umwandlungsprodukte von Cellulose, Hemicellulose und Lignin, die meist wiederum unter hohem Energieaufwand destillativ abgetrennt werden wie z.B. in Organosolv Verfahren. *(*C. Arato, E.K. Pye, G. Gjennestad, 2005, The Lignol approach to biorefining of woody biomass to produce ethanol and chemicals. Appl. Biochem. Biotechnol., Vol. 121-12:871-882*. Pilotprojekt "Lignocellulose-Bioraffinerie" Gemeinsamer Schlussbericht zu den wissenschaftlichen Ergebnissen aller Teilvorhaben*)*.*

Lignin ist ein wichtiger Rohstoff, der aufgrund seiner Struktur als Biowerkstoff', z. B. als Baustein in Duroplasten oder in Kunststoffen oder auch zur Herstellung chemischer Produkte und in der Bioraffeinerie verwendet werden kann, und der beispielsweise aus Biomasse, beispielweise nach deren alkalischem Aufschluss, gewonnen werden kann.

Die Ligninfällung aus dabei erhaltenen alkalischen Lösungen durch Ansäuern ist bekannt. Lignin aus dem alkalischen Aufschluss ist nach Säurefällung allerdings im allgemeinen nichtt filtrierbar, sondern es muß zuerst einem Reifeprozess, bestehend aus Erwärmung und Ruhen lassen, unterzogen werden Wird beispielsweise Lignocellulose durch den Soda-Prozess ohne den Zusatz eines Lösungsmittels, aufgeschlossen (z.B. J.H.Lora & E. Escudero, 2000, Soda pulping of agricultural fibres for boardmaking applications. Paper Technology, May 2000, 37-42*.),* so ist das anfallende Lignin nicht filtrierbar. Zu seiner Abtrennung und Gewinnung wird nachträgliches Erwärmen und Reifen vorgeschlagen *(siehe A. Abaecherli, F. Doppenberg, 2003, Method for preparing alkaline solutions containing aromatic polymers,* EP0970275*).*

Es wurde nunmehr überraschenderweise gefunden, dass Lignin durch Filtration abtrennbar wird, wenn Lignocellulose durch eine alkalische, alkoholische Lösung unter 100°C aufgeschlossen wurde und die Säurefällung des Lignins aus dieser alkalischen, alkoholischen Lösung erfolgt, wobei eine vorherige Aufkonzentrierung, beispielsweise ein vorheriges Abtrennen von Alkohol aus der Lösung, erfolgen kann.

In einem Aspekt stellt die vorliegende Erfindung ein Verfahren zur Gewinnung von Lignin zur Verfügung, das dadurch gekennzeichnet ist, dass einer alkalischen, alkoholischen Lösung von Lignin Säure zugesetzt und das ausgefällte Lignin abgetrennt wird, und aus dem Filtrat zur Gewinnung von weiterem Lignin Alkohol entfernt wird.

Durch die Säurezugabe in einem Verfahren gemäß vorliegender Erfindung wird der pH-Wert der alkoholischen, alkalischen ligninhältigen Lösung, z. B. Aufschlusslösung, durch Zugabe von Säure auf einen pH-Wert zwischen pH 7 und pH 1, bevorzugt zwischen pH 7 und pH 2, besonders bevorzugt zwischen pH 2,5 und 1,5 abgesenkt.

In einem weiteren Aspekt wird in einem Verfahren gemäß vorliegender Erfindung der pH-Wert der alkoholischen, alkalischen Lösung durch Zugabe von Säure stufenweise zwischen pH 7 und pH 1 abgesenkt, wobei nach jeder Stufe der pH Absenkung das ausgefallene Lignin abgetrennt wird, insbesondere abgetrennt durch Zentrifugation, Filtration, Ultrafiltration, insbesondere wobei die erhaltenen Ligninfraktionen getrocknet werden und der, eventuell noch in der/den gewonnenen Ligninfraktionen befindliche Alkohol durch Trocknen zurück gewonnen wird.

Ein Verfahren zur Gewinnung von Lignin, das gemäß vorliegender Erfindung zur Verfügung gestellt wird, wird hierin auch als "Verfahren gemäß (nach) vorliegender Erfindung" bezeichnet.

Es hat sich überraschenderweise gezeigt, dass sich bei einem Verfahren gemäß vorliegender Erfindung Ligninfraktionen mit höherem Molekulargewicht durch den Säurezusatz ausgefällt werden, während Ligninfraktionen mit, im Vegleich zu den ausgfällten Fraktionen niedrigerem Molekulargewicht in Lösung bleiben. Nach dem Entfernen des ausgefällten Ligninfraktion, z. B. durch Abfiltrieren, können durch Entfernen von Alkohol aus der zurückbleibenden Lösung, z.B. dem zurückbleibenden Filtrat, weitere Ligninfraktionen ausgefällt werden. Ein Verfahren gemäß vorliegender Erfindung ist daher besonders geeignet zur Gewinnung von Lignin in fraktionierter Form, das heißt zur Gewinnung von Ligninfraktionen verschiedenen Molekulargewichts.

In einem weiteren Aspekt stellt die vorliegende Erfindung ein Verfahren zur fraktionierten Gewinnung von höher- und niedermolekularem Lignin zu Verfügung, das dadurch gekennzeichnet ist, dass einer alkalischen, alkoholischen Lösung von Lignin Säure zugesetzt, die ausgefällte höhermolekulare Ligninfraktion abgetrennt und aus der, nach dem Abtrennen erhaltenen Lösung Alkohol zur Ausfällung von niedermolekularen Ligninfraktionen entfernt wird, bevorzugt durch Verdampfung, Vakuumverdampfung, Destillation, Vakuumdestillation.

In einem Verfahren gemäß vorliegender Erfindung hat die niedermolekulare Ligninfraktion ein Mn (Number average molecular weight) von 1000 D bis 650 D, bevorzugt 750 D und ein Mw (Weight average molecular weight) von 1500 D bis 1000 D, bevorzugt 1250 D. Die hochmolekulare Ligninfraktion besitzt ein Mn von 1500 D bis 1000 D, bevorzugt 1100 D bis 1200 D, und ein Mw von 7000 D bis 3000 D, bevorzugt 4500 D - 5000 D.

Unter mittlerem Molekulargewicht wird hierin das mittels Agilent-Chemstation-GPC-Data-Analysis-Software berechnete Gewichtsmittel (Mw) und/oder Zahlenmittel (Mn) der Molekulargesichtsverteilung verstanden, wobei die Molekulargewichtsverteilung chromatographisch mittels HPLC unter Verwendung von 3 hintereinander geschalteten TSK-GEL-Säulen (Tosoh G4000PW/G3000PW/G3000PW), 10 mM Natronlauge als mobile Phase, UV-Detektion bei 280 nm, sowie PSS-(Poly(styrensulfonat)-Natriumsalz-)-Kalibrationsstandards im Molekulargewichtsbereich von 0,3 kD bis 35 kD ermittelt wird.

Eine alkalische, alkoholische Lösung von Lignin kann beispielsweise dadurch hergestellt werden, dass lignocellulosisches Material mit einer wässrigen Lösung, welche einen Alkohol, insbesondere einen C₁₋₆-Alkohol, enthält und einen pH-Wert zwischen 11,0 und 14,0 aufweist, behandelt wird, wobei Lignocellulose gespalten wird und wobei ein mit Cellulose und Hemicellulose angereichertes Material erhalten und abgetrennt wird. Ligninfraktionen bleiben dabei überraschenderweise praktisch quantitativ in der Lösung.

Als Alkohol wird bevorzugt ein wasserlöslicher Alkohol eingesetzt, z. B. ein aliphatischer Alkohol, wie ein C₁₋₆Alkohol, oder Mischungen solcher Alkohole, besonders bevorzugt ein C₁₋₄Alkohol, wie Ethanol, Isopropanol. In einer bevorzugten Ausführungsform wird Ethanol, in einer anderen bevorzugten Ausführungsform Isopropanol als Alkohol eingesetzt.

Alkohol liegt in einer wässrigen Lösung beim Aufschluss und im erfindungsgemäßen Verfahren bevorzugt in einem Ausmaß von 10 bis 70 Vol%, z. B. von 30 bis 60 Vol% vor. Es wurde gefunden, dass, um hohe Selektivität des Ligninabbaus zu erreichen etwa 30% Vol%, und um hohe Ligninausbeute zu erreichen, etwa 60 Vol%, eingesetzt werden können.

Der Lignocelluloseaufschluss wird bei einer Temperatur von unter 100°C, wie unter 80°C, z.B. unter 60°C, gespalten, bevorzugt zwischen 40°C bis 90°C, besonders bevorzugt zwischen 50°C bis 70°C.

Die Aufschlussdauer beträgt bevorzugt 2 Stunden bis 36 Stunden, besonders bevorzugt 3 bis 18 Stunden.

Zur Aufspaltung des lignocellulosischen Materials liegt das lignocellulosische Material in der wässrigen alkalischen, alkoholischen Mischung vorzugsweise in einer Stoffdichte von 3-40 Gew%, wie 5-40 Gew%, insbesondere 5-20% Gew%, vor.

Der pH Wert zwischen 11,0 und 14,0 der wässrigen Mischung für die Aufspaltung kann mit einer Base, bevorzugt einer anorganischen Base, wie ein anorganisches Hydroxid, insbesondere ein Hydroxid eines Alkali- oder Erdalkalimetalls, z. B. Kalilauge, Natronlauge, bevorzugt Natronlauge, eingestellt werden.

Die Aufschlußlösung ist zusammengesetzt aus einer Base, einem Alkohol, insbesondere einem C₁₋₄Alkohol, und Wasser. Die Konzentration der Lauge beträgt, bezogen auf das Trockengewicht der aufzuschließenden Lignocellulose, 3 bis 12 Gew%, abhängig vom Lignifizierungsgrad des Substrates, besonders bevorzugt 5 bis 8 Gew%.

Für den Aufschluß wird zerkleinerte Lignocellulose, bevorzugt zerkleinertes Stroh, in einem Reaktionsgefäß mit der vorgewärmten Reaktionslösung gemischt. Der Feststoffgehalt kann bei Beginn des Aufschlussverfahrens zwischen 3-40 Gew% betragen, üblicher Weise aber zwischen 5-20%, z. B. 10-20%.

Als lignocellulosisches Material wird vorzugsweise Stroh, Energiegräser, wie z. B. Switchgrass, Elefantengras oder Abaca, Sisal, Bagasse, oder untypische Lignocellulosesubstrate, wie Spelzen, z.B. Reisspelzen, bevorzugt Stroh, Energiegräser Bagasse oder Spelzen, besonders bevorzugt Stroh oder Bagasse, z. B. Stroh, eingesetzt.

Stroh hat eine stark hydrophobe Oberfläche, sodass die Benetzung mit wässrigen Lösungen ein Problem darstellt. Es hat sich gezeigt, dass es durch die Verwendung von Alkohol möglich ist, selbst ohne Druck die Reaktionslösung in die Poren des Substrates einzubringen und die vorhandene Luft durch Reaktionslösung zu ersetzen. Ferner hat sich gezeigt, dass bei den gewählten Reaktionsbedingungen Alkohol die Extraktion der Spaltprodukte aus Stroh beschleunigt und dazu beiträgt, die Ligninspaltprodukte in Lösung zu halten und dass zusätzlich Alkohol die Löslichkeit der Hemicellulose und deren Spaltprodukte herabsetzt und somit die Hemicellulose im Substrat gehalten wird.

Je nach angestrebter weiterer Verwendung der Produkte kann durch die Kombination der Parameter NaOH-Konzentration, Temperatur, Dauer, Stoffdichte und Alkohol Konzentration entweder eine maximale Ligninausbeute von etwa 90 % bei geringem Hemicelluloseabbau (ca. 10%), oder eine Ligninausbeute von ca 70 % bei unter 1% Hemicelluloseabbau erreicht werden. Die Verwendung von Alkohol erlaubt somit weitgehend die Entkoppelung des Ligninabbaus vom Hemicelluloseabbau.

Durch geeignete Auswahl der Konzentration der Aufschlusskomponenten Alkohol, Wasser und Lauge, sowie der Reaktionsparameter Temperatur, Aufschlussdauer und Feststoffkonzentration für die jeweils aufzuschließende Lignocellulose kann eine angestrebte Ligninausbeute eingestellt werden. Beispielsweise können für die jeweils aufzuschließende Lignocellulose Regressionsmodelle für Aufschlussdaten erstellt werden, aus denen für den jeweils angestrebten Ligningehalt des nach dem Aufschluss anfallenden Feststoffes die optimale prozentuelle Zusammensetzung der Aufschlusskomponenten und die optimalen Reaktionsparameter gewählt werden können. Die Erstellung solcher Regressionsmodelle kann dabei mit statistischer Versuchsplanung erfolgen.

Im Falle der vorwiegend angestrebten Ausrichtung des erfindungsgemäßen Verfahrens als Bioraffinerie Prozess wird das beim Aufschluss gelöste Lignin vom Feststoff abgetrennt. Dies kann z.B. durch Filtration, Zentrifugieren oder Auspressen, beispielsweise mit Hilfe einer Schneckenpresse, erfolgen. Der Feststoff wird weiters einem Waschprozess unterzogen.

Die ligninhältige Aufschlusslösung wird vom Feststoff z.B. durch Filtration oder Zentrifugieren abgetrennt.

In einem weiteren Aspekt stellt die vorliegende Erfindung ein Verfahren zur Gewinnung von Lignin aus lignocellulosischem Material durch Aufschluss mit Alkohol, insbesondere mit einem C₁₋₄Alkohol, insbesondere mit Ethanol, Wasser und einer Lauge, insbesondere Natronlauge, bei einer Temperatur von unter 100°C, insbesondere von 40°C bis 90°C, insbesondere von 50°C bis 70°C, insbesondere, dass die Konzentration der Lauge bezogen auf das Trockengewicht der aufzuschließenden Lignocellulose von 3 bis 12 Gew%, insbesondere von 4 bis 10 Gew%, insbesonders von 5 bis 8 Gew% beträgt, zur Verfügung, das dadurch gekennzeichnet, dass das in der alkalischen, alkoholischen Lösung befindliche Lignin vom Feststoff abgetrennt wird, insbesondere abgetrennt wird durch Pressen, Zentrifugieren, Filtrieren, und das Lignin aus der Aufschlusslösung durch Zugabe einer Säure in Form einer löslichen niederer molekularen und einer unlöslichen höher molekularen Ligninfraktion aufgetrennt wird, insbesondere wobei die höhermolekulare Fraktion durch die Zugabe der Säure ausfällt und anschließend abgetrennt wird und die niedermolekulare Fraktion durch Entfernen des Alkohols aus der Lösung ausgefällt und anschließend abgetrennt wird.

Die Gewinnung des Lignins aus der Aufschlusslösung nach Abtrennung des Feststoffes erfolgt durch Ausfällen des Lignins nach Ansäuern der Lösung.

Dazu gibt es die Möglichkeiten:
1) Ansäuern und Ausfällen aus der Aufschlusslösung ohne Aufkonzentrierung
2) Ansäuern und Ausfällen aus der Aufschlusslösung nach Aufkonzentrierung durch z.B. Eindampfen, (Vakuum)-Destillation, Ultrafiltration, Nanofiltration.

In einem weiteren Aspekt erfolgt in einem Verfahren gemäß vorliegender Erfindung vor der Aufkonzentrierung des Lignins in der alkalisch alkoholischen Lösung durch Säurefällung ein zusätzlicher Aufkonzentrierungsschritt durch Destillation oder Verdampfen oder Vakuumverdampfen oder Ultrafiltration oder Nanofiltration oder einer Kombination derartiger Schritte, insbesondere durch Ultrafiltration oder Nanofiltration.

In einem weiteren Aspekt ist in einem Verfahren gemäß vorliegender Erfindung eine alkalische, alkoholischen Lösung von Lignin eine solche, die durch die Gewinnung von Lignin aus lignocellulosischem Material durch Aufschluss mit Alkohol, insbesondere mit einem C₁₋₄Alkohol, insbesondere mit Ethanol, Wasser und einer Lauge, insbesondere Natronlauge, bei einer Temperatur von unter 100°C, insbesondere von 40°C bis 90°C, insbesondere von 50°C bis 70°C, insbesondere, dass die Konzentration der Lauge bezogen auf das Trockengewicht der aufzuschließenden Lignocellulose von 3 bis 12 Gew%, insbesondere von 4 bis 10 Gew%, insbesonders von 5 bis 8 Gew% beträgt, erhalten wurde und die, insbesondere durch Eindampfen, (Vakuum)-Destillation, Ultrafiltration, Nanofiltration, aufkonzentriert ist.

In einem Verfahren gemäß vorliegender Erfindung wird durch die Nanofiltration 60-95%, bevorzugt 70-95%, besonders bevorzugt 80-95% des in der Aufschlusslösung befindlichen, gelösten Lignins im Retentat zurückgehalten. Das im Retentat aufkonzentrierte Lignin wird durch die Fällungabgetrennt. Beispielsweise wird durch die Aufkonzentrierung des Lignins bei der Nanofiltration im Retentat und durch die erzielte Volumsreduzierung des Retentats die Menge der zur Fällung erforderlichen Säure um 50-90%, bevorzugt um 70-90, besonders bevorzugt um 80-90% reduziert. Das Permeat, welches eine Menge der Aufschlusslösung von 60-90%, bevorzugt con 70.90%, besonders bevorzugt von 80-90% ausmacht und nur Restmengen an Lignin enthält, wird in den Aufschluss zurückgeführt. Das zurückgeführte Permeat wird einem weiteren Reinigungsschritt, insbesondere einer Umkehrosmose, Destillation, Ausfällung von Salzen, Ionenaustauscherbehandlung unterzogen.

Variante 1) hat den Nachteil, dass hohe Konzentrationen von Säuren erforderlich sind um die alkalische Aufschlusslösung von ca. pH 11 auf pH 7 - pH 1 zu senken. Dies trifft auch auf Variante 2 mit Aufkonzentrierung durch Eindampfen oder (Vakuum)-Destillation zu, da die Konzentration der OH-Ionen in der Lösung dadurch nicht verändert wird.

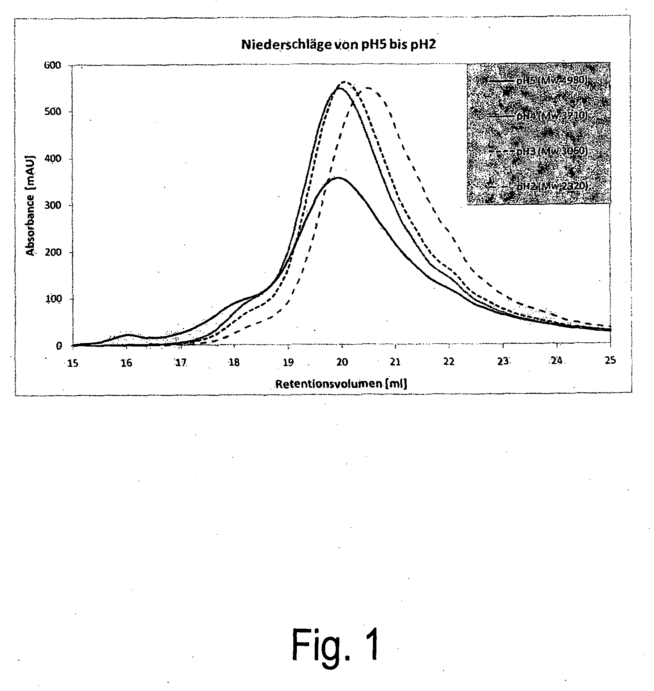

Es hat sich aber überraschenderweise gezeigt, dass eine Ultra- und/oder Nanofiltration zur Aukonzentration besonders vorteilhaft ist. Denn bei der Aufkonzentrierung des Lignins durch Ultra- und/oder Nanofiltration befinden sich, je nach Ausmaß der Aufkonzentrierung, beispielsweise etwa 80% der OH-Ionen im Permeat und werden in den Aufschluss rückgeführt und nur 20% bleiben pH-wirksam.
Es hat sich weiters gezeigt, dass mit abnehmendem pH-Wert auch das Molekulargewicht der gewonnen Ligninfraktion sinkt. Eine typische Molekulargewichtsverteilung ist in der Abbildung in Fig. 1 dargestellt.

Die Ultra- und oder Nanofiltration zur Aufkonzentrierung des Lignins in einer alkalischen, alkoholischen Lösung ist neu und ebenfalls Gegenstand der vorliegenden Erfindung.

In einem weiteren Aspekt stellt die vorliegende Erfindung ein Verfahren zur Herstellung einer aufkonzentrierten, alkalischen, alkoholischen Lösung von Lignin zur Verfügung, die dadurch gekennzeichnet ist, dass eine alkalische, alkoholische Lösung von Lignin, insbsondere eine solche, die nach einem der oben beschriebenen Aufschlussverfahren erhalten wurde, einer Membranfiltration, wie einer Ultra- und oder Nanofiltration unterzogen wird, wobei gegebenenfalls Alkohol teilweise entfernt wird.

Gegebenenfalls wird das Permeat vor der Rückführung in den Aufschlussprozess einem weiteren Reinigungsschritt oder mehrer Reinigungsschritte, insbesondere einer Umkehrosmose, Destillation, Ausfällung von Salzen, Ionenaustauscherbehandlung unterzogen.

Erfolgt die Aufkonzentrierung durch Membranfiltration und durch teilweise Entfernung von Alkohol, so können diese beiden Schritte in beliebiger Reihenfolge durchgeführt werden, bevorzugt wird jedoch die Aufkonzentrierung des Lignins durch Membranfiltration mit nachfolgender Abtrennung des Alkohols, da bei der Membranfiltration eine Volumsreduktion der Lösung auf ca. 20% erfolgt und die für die Abtrennung des Alkohols aufzuwendende (thermische) Energie dadurch stark reduziert wird. Weiters enthält die rückgeführte Lösung bereits wieder Ethanol für einen weiteren Aufschluss.

Die Aufkonzentrierung des Lignins durch teilweises Entfernen des Alkohols darf nur so weit erfolgen, dass das niedermolekulare Lignin dabei in Lösung gehalten wird. Bei vollständiger Entfernung des Alkohols ist eine Trennung des Lignins in eine beim Ansäuern in Lösung bleibende niedermolekulare und eine ausfallende hochmolekulare Fraktion nicht mehr möglich, da das gesamte Lignin ausfällt.

Die Ausfällung des Lignins aus dem Retentat der (Ultra-) / Nanofiltration kann durch Ansäuern der Lignin enthaltenden Lösung in einem Schritt erfolgen, oder kann in einzelnen Fraktionen, abgestuft in verschiedenen pH-Wert Stufen von pH = 7 bis pH = 1 erfolgen. Dies trifft sowohl für eine Alkohol enthaltende Ligninlösung zu, wie auch für eine Ligninlösung, aus der der Alkohol entfernt worden war, zu.

Zum Ansäuern in einem Verfahren gemäß vorliegender Erfindung kann eine anorganische oder organische Säure verwendet werden. Bevorzugt wird eine anorganische Säure, beispielsweis H₂SO₄, H₃PO₄, HCl verwendet.

Die Gegenwart von Alkohol in der Ligninlösung hat einen wesentlichen Einfluss auf die Fällbarkeit bestimmter Anteile der Ligninfraktion: Erfolgt die Fällung ohne vorherige Entfernung des Alkohols durch Abdampfen oder (Vakuum)-Destillation, so werden dabei nur etwa 50% des Lignins gefällt, während die andere Hälfte in Lösung bleibt.

Die Bestimmung des durchschnittlichen Molekulargewichtes der beiden Fraktionen, die wie oben angegeben durchgeführt wurde, hat gezeigt, dass die ausgefällte Fraktion ein mittleres Mn von 1150 D und ein Mw von 5000 D aufweist, also hochmolekular ist, während die lösliche Fraktion mit einem mittleren Mn von 750 D und einem Mw von 1250 niedermolekular ist.

Die lösliche Fraktion wird durch Entfernen des Alkohols, gegengebenenfalls fraktioniert, ausgefällt.

Das gemäß dem erfindungsgemäßen Verfahren gewonnene Lignin bzw. die einzelnen Ligninfraktionen können zur Abtrennung von Verunreinigungen wie z. B. Salzen, niedermolekularen Verbindungen mit geeigneten Lösungsmitteln, z. B. Wasser oder organischen Lösungsmitteln, in denen die Ligninfraktionen nicht löslich sind, gewaschen werden.

Die jeweils erhaltenen Ligninfraktionen können getrocknet werden. Der, eventuell noch in der/den gewonnenen Ligninfraktionen befindliche Alkohol kann dabei rückgewonnen werden.

Das gemäß dem erfindungsgemäßen Verfahren gewonnene Lignin wird bevorzugt zu höherwertigen Produkten, insbesondere Phenolharzen, Treibstoffkomponenten, Carbonfasern, Kunststoff-Copolymeren oder pharmazeutischen Produkten weiterverarbeitet.

Die Weiterverarbeitung kann in geeigneter Weise, z. B. analog bekannter Lignin-Weiterververarbeitungsverfahren erfolgen. Beispielsweise kann das gewonnene niedermolekulare Lignin zu Produkten verarbeitet werden, die ein niedriges Molekulargewicht erfordern, wie z.B. Lignin Derivate mit Molekulargewicht bis um die 1000 z.B. zur Herstellung von Harzen für die Erzeugung von Hochdruckschichtpressstoffen wie Compactplatten und Laminaten und das höhermolekulare Lignin kann zu Produkten verarbeitet werden, die ein Molekulargewicht erfordern, welches wesentlich höher als 1000 ist, wie z.B. zur Herstellung von Harzen, die zur Erzeugung von Holzfaserplatten verwendet werden.

Im flüssigen Überstand der Ligninfällungen befindliche wasserlösliche Stoffe, insbesondere Säuren, säurelösliche Ligninanteile, Salze können nach Isolieren des ausgefällten Lignins aus der erhaltenen Lösung in weiteren Schritten abgetrennt werden. Diese Vorgangsweise eignet sich im Besonderen zur Abtrennung der niedermolekularen Hydroxyzimtsäuren, wie Ferulasäure und Coumarsäure, die durch zusätzliche Trennverfahren, z. B. Adsorption an Trägermaterialien oder flüssig/flüssig Extraktion mit geeigneten Lösungsmitteln abgetrennt werden können.

Eine weitere Möglichkeit besteht darin, das PVPP in einer gepackten Säule einzusetzen, durch die die Lösung, die Hydroxyzimtsäuren enthält, geleitet wird. Dabei können höhere Beladungen erzielt werden und das Verhältnis von eingesetztem PVPP zur aufzureinigenden Flüssigkeitsmenge liegt bei ca. 1:500 oder höher.

In einem weiteren Aspekt stellt die vorliegende Erfindung ein Verfahren gemäß vorliegender Erfindung zur Verfügung, das dadurch gekennzeichnet ist, dass nach Abtrennung des Lignins die verbleibende saure Lösung zur Gewinnung von Hydroxyzimtsäuren aus der Lösung mit Polyvinylpolypyrrolidon behandelt wird, wobei Hydroxyzimtsäuren an das Polyvinylpolypyrrolidon adsorbiert und aus der Lösung entfernt werden.

Die Möglichkeit einer einfachen Trennung der Ligninfraktion in eine hoch- und eine niedermolekulare Fraktion, für die es gesonderte Anwendungsbereiche bestehen, ist ein wesentlicher Vorteil eines Verfahrens gemäß vorliegender Erfindung.

## Patentansprüche

1. Verfahren zur Gewinnung von Lignin, **dadurch gekennzeichnet, dass** einer alkalischen, alkoholischen Lösung von Lignin Säure zur Einstellung eines pH-Wertes zwischen pH 7 und pH 1 zugesetzt und das ausgefällte Lignin abgetrennt und aus dem Filtrat zur Gewinnung von weiterem Lignin Alkohol entfernt wird.

2. Verfahren nach Anspruch 1, **dadurch gekennzeichnet, dass** einer alkalischen, alkoholischen Lösung von Lignin Säure zugesetzt, die ausgefällte höhermolekulare Ligninfraktion abgetrennt und aus der, nach dem Abtrennen erhaltenen Lösung Alkohol zur Ausfällung von niedermolekularen Ligninfraktionen entfernt wird, insbesondere, durch Verdampfung, Vakuumverdampfung, Destillation, Vakuumdestillation.

3. Verfahren nach einem der Ansprüche 1 oder 2, zur Gewinnung von Lignin aus lignocellulosischem Material durch Aufschluss mit Alkohol, insbesondere mit einem C₁₋₄Alkohol, insbesondere mit Ethanol, Wasser und einer Lauge, insbesondere Natronlauge, bei einer Temperatur von unter 100°C, insbesondere zwischen 40°C bis 90°C, insbesondere zwischen 50°C bis 70°C, insbesondere, dass die Konzentration der Lauge, bezogen auf das Trockengewicht der aufzuschließenden Lignocellulose zwischen 3 bis 12 Gew%, insbesondere zwischen 4 bis 10 Gew%, insbesonders zwischen 5 bis 8 Gew% beträgt, **dadurch gekennzeichnet, dass** das in der alkalischen, alkoholischen Lösung befindliche Lignin vom Feststoff abgetrennt wird, insbesondere abgetrennt wird durch Pressen Zentrifugieren, Filtrieren, und das Lignin aus der Aufschlusslösung durch Zugabe einer Säure in Form einer löslichen niederermolekularen und einer unlöslichen höhermolekularen Ligninfraktion aufgetrennt wird, insbesondere wobei die höhermolekulare Fraktion durch die Zugabe der Säure ausfällt und anschließend abgetrennt wird und die niedermolekulare Fraktion durch Entfernen des Alkohols aus der Lösung ausgefällt und anschließend abgetrennt wird.

4. Verfahren nach einem der Ansprüche 1 bis 3, **dadurch gekennzeichnet, dass** das lignocellulosische Substrat Stroh, insbesondere Weizenstroh ist.

5. Verfahren nach einem der Ansprüche 2 bis 4, **dadurch gekennzeichnet, dass** das mittlere Molekulargewicht der niedermolekularen Ligninfraktion ein Mn (Number average molecular weight) von 1000 D bis 650 D, insbesondere 750 D und ein Mw (Weight average molecular weight) von 1500 D bis 1000 D, insbesondere 1250 D; und das mittlere Molekulargewicht der hochmolekularen Ligninfraktion ein Mn von 1500 D bis 1000 D, insbesondere 1100 D bis 1200 D, und ein Mw von 7000 D bis 3000D, insbesondere 4500 D bis 5000 D beträgt.

6. Verfahren nach einem der Ansprüche 1 bis 5, **dadurch gekennzeichnet, dass** der pH-Wert der alkoholischen, alkalischen Lösung, insbesondere Aufschlusslösung, durch die Zugabe von Säure auf einen pH-Wert zwischen pH 7 und pH 1, insbesondere zwischen pH 7 und pH 2, insbesondere auf (etwa) pH 2 abgesenkt wird.

7. Verfahren nach einem der Ansprüche 1 bis 6, **dadurch gekennzeichnet, dass** der pH-Wert der alkoholischen, alkalischen Lösung durch Zugabe von Säure stufenweise zwischen pH 7 und pH 1 abgesenkt wird, wobei nach jeder Stufe der pH Absenkung das ausgefallene Lignin abgetrennt wird, insbesondere abgetrennt durch Zentrifugation, Filtration, Ultrafiltration, insbesondere wobei die erhaltenen Ligninfraktionen getrocknet werden und der, eventuell noch in der/den gewonnenen Ligninfraktionen befindliche Alkohol durch Trocknen zurück gewonnen wird.

8. Verfahren nach einem der Ansprüche 1 bis 7, **dadurch gekennzeichnet, dass** vor der Aufkonzentrierung des Lignins in der alkalisch alkoholischen Lösung durch Säurefällung ein zusätzlicher Aufkonzentrierungsschritt durch Destillation oder Verdampfen oder Vakuuumverdampfen oder Ultrafiltration oder Nanofiltration oder einer Kombination derartiger Schritte, insbesondere durch Ultrafiltration oder Nanofiltration, erfolgt.

9. Verfahren zur Herstellung einer aufkonzentrierten, alkalischen, alkoholischen Lösung von Lignin, die **dadurch gekennzeichnet ist, dass** eine alkalische, alkoholische Lösung von Lignin, die nach einem der in vorigen Ansprüchen beschriebenen Aufschlussverfahren erhalten wurde, einer Membranfiltration, wie einer Ultra- und oder Nanofiltration unterzogen wird, wobei Alkohol teilweise entfernt wird.

10. Verfahren gemäß einem der vorherigen Ansprüche, **dadurch gekennzeichnete dass** das gewonnene niedermolekulare Lignin zu Produkten verarbeitet wird, die ein niedriges Molekulargewicht erfordern, insbesondere zur Herstellung von Harzen für die Erzeugung von Hochdruckschichtpressstoffen wie Compactplatten und Laminaten; und das höhermolekulare Lignin zu Produkten verarbeitet wird, die ein höheres Molekulargewicht erfordern, insbesondere zur Herstellung von Harzen, zur Erzeugung von Holzfaserplatten.

11. Verfahren gemäß einem der Ansprüche 1 bis 8, **dadurch gekennzeichnet, dass** nach Abtrennung des Lignins die verbleibende saure Lösung zur Gewinnung von Hydroxyzimtsäuren aus der Lösung mit Polyvinylpolypyrrolidon behandelt wird, wobei Hydroxyzimtsäuren an das Polyvinylpolypyrrolidon adsorbiert und aus der Lösung entfernt werden.

## Claims

1. A method for the preparation of lignin, **characterized in that** an acid is added to an alkaline, alcoholic solution of lignin for adjusting a pH value to between pH 7 and pH 1 and the precipitated lignin is separated, and from the filtrate alcohol is removed in order to get further lignin.

2. A method according to claim 1, **characterized in that** an acid is added to an alkaline, alcoholic solution of lignin, the precipitated higher-molecular lignin fraction is separated and from the solution obtained upon separation alcohol is removed, in particular by vaporization, vacuum vaporization, distillation, vacuum distillation, in order to precipitate low-molecular lignin fractions.

3. A method according to any of claims 1 or 2 for preparing lignin from lignocellulosic material by pulping with alcohol, in particular a C₁₋₄ alcohol, in particular ethanol, water and a base, in particular sodium hydroxide, at a temperature below 100°C, in particular from 40°C to 90°C, in particular from 50°C to 70°C, in particular such that the concentration of the base, based on the dry weight of the lignocellulose to be pulped, is from 3 to 12% by weight, in particular from 4 to 10% by weight, in particular from 5 to 8% by weight, **characterized in that** the lignin present in the alkaline, alcoholic solution is separated from the solid, in particular separated by pressing, centrifugation, filtration, and the lignin is separated from the pulping solution by addition of an acid in the form of a soluble, lower-molecular and an insoluble higher-molecular lignin fraction, in particular wherein the higher-molecular fraction is precipitated by addition of the acid and is subsequently separated, and the low-molecular fraction is precipitated by removal of the alcohol from the solution and is subsequently separated.

4. A method according to any of claims 1 to 3, **characterized in that** the lignocellulosic substrate is straw, in particular wheat straw.

5. A method according to any of claims 2 to 4, **characterized in that** the average molecular weight of the low-molecular lignin fraction has an Mn (number average molecular weight) of 1000 D to 650 D, in particular 750 D, and an Mw (weight average molecular weight) of 1500 D to 1000 D, in particular 1250 D; and the average molecular weight of the high-molecular lignin fraction has an Mn of 1500 D to 1000 D, in particular 1100 D to 1200 D, and an Mw of 7000 D to 3000 D, in particular 4500 D to 5000 D.

6. A method according to any of claims 1 to 5, **characterized in that** the pH of the alcoholic, alkaline solution, in particular pulping solution, is decreased by addition of an acid to a pH value of between pH 7 and pH 1, in particular of between pH 7 and pH 2, in particular to (about) pH 2.

7. A method according to any of claims 1 to 6, **characterized in that** the pH value of the alcoholic, alkaline solution is decreased step-by-step by addition of an acid between pH 7 and pH 1, wherein the precipitated lignin is separated, in particular separated by centrifugation, filtration, ultra-filtration, following each step of the pH decrease, in particular wherein the lignin fractions obtained are dried and the alcohol possibly still present in the lignin fraction/s obtained is recovered by drying.

8. A method according to any of claims 1 to 7, **characterized in that** before concentrating the lignin in the alkaline, alcoholic solution by acid precipitation an additional concentration step by distillation, or vaporization, or vacuum vaporization, or ultra-filtration, or nano-filtration, or a combination of such steps, in particular by ultra-filtration, or nano-filtration, is carried out.

9. A method for the preparation of a concentrated, alkaline, alcoholic solution of lignin, **characterized in that** an alkaline, alcoholic solution of lignin, which has been obtained according to any of the pulping methods described in preceding claims, is subjected to a membrane filtration such as an ultra- and/or nano-filtration, whereby alcohol is removed partially.

10. A method according to any of the preceding claims, **characterized in that** the low-molecular lignin obtained is processed into products requiring a low molecular weight, in particular for the preparation of resins for the production of high-pressure laminates like compact laminates and laminates; and the higher-molecular lignin is processed into products requiring a higher molecular weight, in particular for the preparation of resins for the production of wood fibre boards.

11. A method according to any of claims 1 to 8, **characterized in that**, following the separation of the lignin, the remaining acid solution is treated with polyvinyl polypyrrolidone in order to isolate hydroxy cinnamic acids from the solution, which hydroxy cinnamic acids are adsorbed to the polyvinyl polypyrrolidone and removed from the solution.

## Revendications

1. Procédé d'extraction de lignine, **caractérisé en ce qu'**une solution de lignine alcaline à base d'alcool est mélangée à un acide afin d'ajuster une valeur pH entre un pH de 7 et un pH de 1, **en ce que** la lignine précipitée est séparée, et **en ce que** l'alcool est supprimé du filtrat afin d'extraire une lignine supplémentaire.

2. Procédé selon la revendication 1, **caractérisé en ce qu'**est ajouté à la solution de lignine alcaline à base d'alcool, un acide, **en ce que** la fraction de lignine précipitée à poids moléculaire élevé est séparée, et **en ce que** l'alcool est supprimé de la solution obtenue après la séparation afin de précipiter des fractions de lignine à faible poids moléculaire, en particulier par évaporation, par évaporation sous vide, par distillation, par distillation sous vide.

3. Procédé selon l'une quelconque des revendications 1 ou 2 d'extraction de lignine à partir d'un matériau lignocellulosique par dissolution avec un alcool, en particulier avec un alcool en C₁₋₄, en particulier avec de l'éthanol, de l'eau et une lessive, en particulier de la soude caustique, à une température inférieure à 100 °C, en particulier à une température comprise entre 40 °C et 90 °C, en particulier comprise entre 50 °C et 70 °C, en particulier en ce que la concentration de la lessive par rapport au poids sec de la lignocellulose à dissoudre présente une valeur comprise entre 3 et 12 % en poids, en particulier entre 4 et 10 % en poids, en particulier entre 5 et 8 % en poids, **caractérisé en ce que** la lignine se trouvant dans la solution alcaline à base d'alcool est séparée de la matière solide, en particulier est séparée par pressage, centrifugation, filtration, et **en ce que** la lignine issue de la solution de dissolution par ajout d'un acide est décomposée sous la forme d'une fraction de lignine soluble à faible poids moléculaire et en une fraction de lignine non soluble à poids moléculaire élevé, en particulier sachant que la fraction à poids moléculaire élevé est précipitée par l'ajout de l'acide avant d'être immédiatement après séparée et que la fraction à faible poids moléculaire est précipitée de la solution par suppression de l'alcool avant d'être immédiatement après séparée.

4. Procédé selon l'une quelconque des revendications 1 à 3, **caractérisé en ce que** le substrat lignocellulosique est de la paille, en particulier de la paille de blé.

5. Procédé selon l'une quelconque des revendications 2 à 4, **caractérisé en ce que** le poids moléculaire moyen de la fraction de lignine à faible poids moléculaire présente une valeur de Mn (Number average molecular weight - nombre de masse moléculaire moyenne) allant de 1000 D à 350 D, en particulier de 750 D, et une valeur de Mw (Weight average molecular weight - poids de masse moléculaire moyenne) allant de 1500 D à 1000 D, en particulier de 1250 D, et **en ce que** le poids moléculaire moyen de la fraction de lignine à poids moléculaire élevé présente une valeur de Mn allant de 1500 D à 1000 D, en particulier allant de 1100 D à 1200 D, et une valeur de Mw allant de 7000 D à 3000 D, en particulier allant de 4500 D à 5000 D.

6. Procédé selon l'une quelconque des revendications 1 à 5, **caractérisé en ce que** la valeur pH de la solution à base d'alcool alcaline, en particulier de la solution de dissolution, est abaissée par l'ajout d'acide de manière à s'établir à une valeur pH comprise entre un pH de 7 et un pH de 1, en particulier entre un pH de 7 et un pH de 2, en particulier de manière à s'établir à (environ) un pH de 2.

7. Procédé selon l'une quelconque des revendications 1 à 6, **caractérisé en ce que** la valeur pH de la solution à base d'alcool alcaline est abaissée par l'ajout d'acide par phase à une valeur pH comprise entre un pH 7 et un pH de 1, sachant qu'après chaque phase consistant à abaisser le pH, la lignine précipitée est séparée, en particulier est séparée par centrifugation, filtration, ultrafiltration, en particulier sachant que les fractions de lignine obtenues sont séchées et que l'alcool se trouvant éventuellement encore dans la ou les fractions de lignine extraites est à nouveau extrait par séchage.

8. Procédé selon l'une quelconque des revendications 1 à 7, **caractérisé en ce qu'**une étape supplémentaire d'augmentation de la concentration est effectuée par distillation ou par évaporation ou par évaporation sous vide ou par ultrafiltration ou par nanofiltration ou par une combinaison d'étapes de ce type, en particulier par ultrafiltration ou nanofiltration avant l'augmentation de concentration de la lignine dans la solution alcaline à base d'alcool par précipitation acide.

9. Procédé de production d'une solution de lignine alcaline à base d'alcool et à concentration augmentée, qui est **caractérisée en ce qu'**une solution de lignine alcaline à base d'alcool, qui a été obtenue selon un procédé de dissolution décrit dans les précédentes revendications, est soumise à une filtration sur membrane, telle qu'une ultrafiltration ou une nanofiltration, sachant que l'alcool est en partie supprimé.

10. Procédé selon l'une quelconque des revendications précédentes, **caractérisé en ce que** la lignine extraite à faible poids moléculaire est transformée en des produits qui requièrent un faible poids moléculaire, en particulier afin de produire des résines en vue d'obtenir des stratifiés à haute pression tels que des panneaux compacts et des laminés ; et **en ce que** la lignine à poids moléculaire élevée est transformée en des produits, qui requièrent un poids moléculaire plus élevé, en particulier afin de produire des résines, afin d'obtenir des panneaux de fibres de bois.

11. Procédé selon l'une quelconque des revendications 1 à 8, **caractérisé en ce que** le résidu de solution acide est, après la séparation de la lignine, traité avec du polyvinylpolypyrrolidone afin d'extraire des acides hydroxycinnamiques de la solution, sachant que des acides hydroxycinnamiques sont absorbés au niveau du polyvinylpolypyrrolidone et sont supprimés de la solution.
